# EUROPEAN PATENT SPECIFICATION

(11) **EP 2 552 913 B1**
(45) Date of publication and mention of the grant of the patent: **03.08.2016**
(21) Application number: 11716089.5
(22) Date of filing: 23.02.2011
(51) Int. Cl.: C07D 451/10

(54) **METHOD FOR SYNTHESIS OF TIOTROPIUM BROMIDE**
VERFAHREN ZUR SYNTHESE VON TIOTROPIUMBROMID
PROCÉDÉ POUR LA SYNTHÈSE DE BROMURE DE TIOTROPIUM

(30) Priority: 28.06.2010 TR 201005222; 01.04.2010 TR 201002520
(43) Date of publication of application: 06.02.2013
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2011/000052
(87) International publication number: WO 2011/123080

(56) References cited:
- EP-A1- 0 418 716
- WO-A1-03/057694
- WO-A1-2008/089852
- WO-A1-2009/087419
- VERDEGAAL C H M ET AL: "A convenient synthesis of 2'-O-acetal-N2-acyl derivatives of riboguanosine", CHEMISCH WEEKBLAD, SIGMA CHEMIE, DEN HAAG, NL, vol. 100, no. 5, 1 May 1981 (1981-05-01), pages 200-204, XP009148199, ISSN: 0009-2932
- DIAFI ET AL: "8-Hydroxylated derivatives of diazabicyclo[4.3.0]nonanes, 2,5-dioxodiazabicyclo[4.3.0]nonanes and some related esters", JOURNAL OF HETEROCYCLIC CHEMISTRY, vol. 27, 1990, pages 2181-2187, XP002636709, DOI: 10.1002/jhet.5570270758
- HARRY H. WASSERMAN AND BRUCE H. LIPSHUTZ: "Reactions of lithium enolates with molecular oxygen alpha-hydroxylation of amides and other carboxylate derivatives", TETRAHEDRON LETTERS, vol. 16, 1975, pages 1731-1734, XP002614576, DOI: 10.1016/S0040-4039(00)72245-3

## Description

The present invention relates to a new method for producing (1α, 2β, 4β, 5α, 7β)-7-[(hydroxidi-2-thienylacetyl)oxy]-9,9-dimethyl-3-oxa-9- azoniatricyclo[3.3.1.0^{2,4}]nonane-bromide.

### Background of the Invention:

The compound which has the chemical name (1α, 2β, 4β, 5α, 7β)-7-[(hydroxidi-2-thienylacetyl)oxy]-9,9-dimethyl-3-oxa-9- azoniatricyclo[3.3.1.0^{2,4}]nonane-bromide is generally known as tiotropium bromide. This compound given in the formula 6 below was first disclosed in the patent numbered EP 418716.

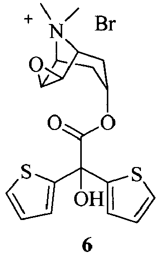

Tiotropium bromide is a highly effective anticholinergic agent. Due to this reason, it is commonly utilized in the treatment of asthma and COPD (chronic obstructive lung disease).

Tiotropium bromide is preferably administered to patients by the inhalation route. For the administration by the inhalation route, dry powder inhalers in which the dry powder is stored in capsules or blisters can be used. Another method comprises the administration of tiotropium bromide to patients in aerosol form with various gases (e.g. HFA134a and/or HFA227).

As tiotropium bromide is highly effective, even small amounts of it present therapeutic effects.

The patent numbered EP 418716 discloses a synthesis method for the preparation of tiotropium bromide demonstrated in Scheme 1.

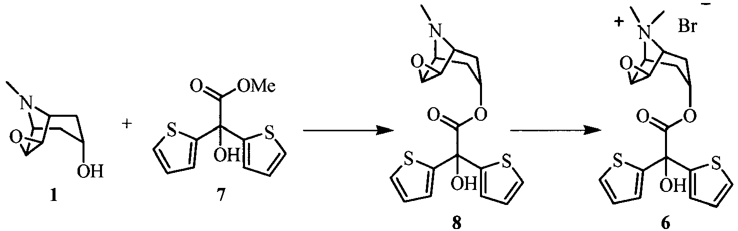

### Scheme 1:

According to this method, scopine that is demonstrated in formula 1 at the first stage reacts with di-(2-thienyl)-gylicolic acid methyl ester that is demonstrated in formula 6 and transforms into (2-thienyl)-gylicol acid scopine ester shown in formula 7. Subsequently, tiotropium bromide is obtained by quaternizing the compound demonstrated in formula 7.

The first stage of this two-stage synthesis method is generally carried out at high temperatures like 70-90°C with hazardous chemicals such as sodium methoxide and metallic sodium. It is not preferable for manufacturers to carry out the reaction at high temperatures as it increases the cost. In addition, the efficacy of the reaction is in the range of 45-70% although it is realized under such tough conditions.

Additionally, WO2009/087419 and US2003232993 disclose synthesis methods for scopine ester which is used in the preparation of pure tiotropium as a primary intermediate and WO2008/089852 relates to the synthesis of tiotropium salts.

All these indicate that synthesis methods with higher efficacy are needed in tiotropium bromide synthesis.

### Detailed Explanation of the Invention

Surprisingly, it has been found that an easier and more effective obtain of tiotropium bromide is realized compared to the methods of tiotropium bromide in the prior art when the synthesis method pertaining to the present invention is applied. This alternative synthesis method comprises that;
di-(2-thienyl)-acetic acid shown in formula 1 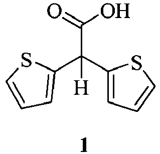 is transformed into formula 2 which is a form of acid anhydride; 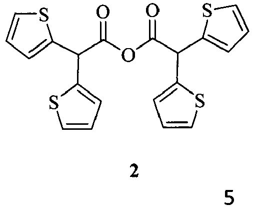 scopine ester that is demonstrated in formula 4 is obtained through reacting the obtained acid anhydride with scopine; 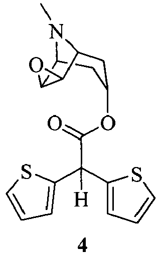 the compound shown in formula 5 is obtained upon quaternizing scopine ester with methyl bromide; 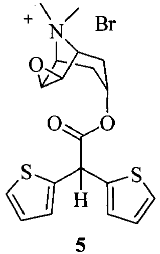 the compound obtained at the end is transformed into tiotropium bromide after it is processed with organic and/or inorganic basic substances.

Steiglich esterification, which is a commonly utilized method to synthesize esters by using alcohol and carboxylic acid, comprises the use of a chemical called N,N'-dicyclohexylcarbodiimide (DCC). However, there appears some problems in the purification of the final product obtained since dicyclohexylurea is produced at the end of the reaction as a byproduct of this chemical.

According to this, a method so as to be used in the synthesis of scopine ester shown in formula 4 which is a significant intermediate product for the synthesis of tiotropium bromide 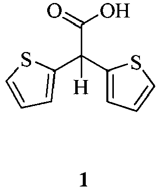 comprises the steps of obtaining di-(2-thienyl)-acetic acid anhydride demonstrated in formula 2 through the reaction of di-(2-thienyl)-acetic acid (1) with DCC; 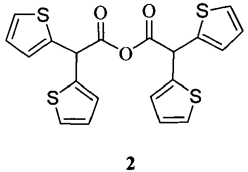 then obtaining scopine ester shown in formula 4 through the reaction of the compound demonstrated in formula 2 and scopine with 4-dimethylaminopyridine (DMAP).

The inventors have found that dicyclohexylurea, which is the byproduct of DCC, is eliminated at the previous stage of the synthesis, and therefore purer scopine ester (4) is obtained.

According to this, the method for the synthesis of di-(2-thienyl)-acetic acid anhydride shown in formula 2 comprises the steps of dissolving di-(2-thienyl)-acetic acid in an appropriate organic solvent such as dimethylformamide (DMF), dimethyl sulfoxide (DMSO), benzene, toluene, diethyl ether, tetrahydrofurane, ethanol, methanol, acetonitrile, acetone, ethyl acetate, methyl ethyl ketone, dichloromethane, dioxane, dimethylacetamide, N-methyl pyrrolidone, hexane, heptane or a combination thereof, most preferably in dichloromethane or dimethylformamide; then adding dicyclohexylcarbodiimide (DCC) into the obtained solution; and stirring it at 0-40°C, preferably at room temperature for 10-48 hours preferably for 12-18 hours.

In said reaction, the amount of DCC added per mole of di-(2-thienyl)-acetic acid used is at least 0,5 mole, preferably in the range of 0,55-3 moles, most preferably in the range of 0,6-1,5 moles.

The solid precipitate which comes out at the end of the reaction is separated through filtration and the solvent in the solution, which is obtained after the solid substance is separated, is removed under convenient conditions. The conditions to be used here, such as temperature and pressure, are determined according to the type of the solvent used.

Another constituent of the invention is to develop a new synthesis method for the preparation of scopine ester shown in formula 4.

According to this, the method for the preparation of scopine ester shown in formula 4 comprises the steps of dissolving scopine and di-(2-thienyl)-acetic acid anhydride in an appropriate organic solvent such as dimethylformamide (DMF), dimethyl sulfoxide (DMSO), benzene, toluene, diethyl ether, tetrahydrofurane, ethanol, methanol, acetonitrile, acetone, ethyl acetate, methyl ethyl ketone, dichloromethane, dioxane, dimethylacetamide, N-methyl pyrrolidone, hexane, heptane or a combination thereof, most preferably in dichloromethane or dimethylformamide; then adding DMAP into the obtained solution; and stirring it at 0-40°C, preferably at room temperature for 10-48 hours preferably for 12-18 hours.

In said reaction, the amount of di-(2-thienyl)-acetic acid anhydride used per mole of scopine used is at least 1 mole, preferably in the range of 1,05-4 moles, most preferably in the range of 1,1-3,0 moles.

In said reaction, the amount of DMAP added per mole of scopine used is at least 0,05 mole, preferably in the range of 0,1-2,0 moles, most preferably in the range of 0,3-1,0 mole.

After the reaction, the amount of distilled water added into the mixture per mole of di-(2-thienyl)-acetic acid anhydride is at least 0,01 mole, preferably in the range of 0,02-0,2 mole, most preferably in the range of 0,05-0,1 mole. After the distilled water is added, the mixture is stirred at room temperature for 2 hours.

The obtained reaction mixture is diluted by an appropriate organic solvent, for example by DMF, DMSO, benzene, toluene, diethyl ether, tetrahydrofurane, ethanol, methanol, acetonitrile, acetone, ethyl acetate, methyl ethyl ketone, dichloromethane, dioxane, dimethylacetamide, N-methyl pyrrolidone, hexane, heptane or a combination thereof, most preferably by dichloromethane or dimethylformamide; and it is extracted with aqueous solutions 1M NaHSO₄, 10% Na₂CO₃ and saturated NaCl respectively. The accumulated solvent layer is dried with and anhydrous Na₂SO₄ filtered. The organic solvent in the solution obtained is removed under convenient temperature and pressure. The conditions to be used here are determined according to the type of the solvent used.

If required, the obtained substance can be purified through one of the conventional purification methods such as anti-solvent crystallization, activated charcoal crystallization, thin layer chromatography (TLC), column chromatography, high pressure liquid chromatography (HPLC), distillation and the methods in the prior art.

The method for the preparation of quaternized scopine ester which is illustrated in formula 5 is comprised of the stages of dissolving scopine ester demonstrated in formula 4 and the organic solution which contains 10-90%, preferably 30-60% methylbromide in an appropriate solvent, for example in a solvent that is comprised of dimethylformamide (DMF), dimethyl sulfoxide (DMSO), benzene, toluene, diethyl ether, tetrahydrofurane, ethanol, methanol, acetonitrile, acetone, ethyl acetate, methyl ethyl ketone, dichloromethane, dioxane, dimethylacetamide, N-methyl pyrrolidone, hexane, heptane or a combination thereof, and stirring the obtained mixture at 0-40 °C, preferably at room temperature for 12-90 hours, preferably for 18-72 hours.

The methyl bromide solution mentioned here is obtained through condensing methyl bromide gas at low temperature, then dissolving it in a desired organic solvent, for instance in a solvent combination which is comprised of dimethylformamide (DMF), dimethyl sulfoxide (DMSO), benzene, toluene, diethyl ether, tetrahydrofurane, ethanol, methanol, acetonitrile, acetone, ethyl acetate, methyl ethyl ketone, dichloromethane, dioxane, dimethylacetamide, N-methyl pyrrolidone, hexane, heptane or a combination thereof. Preferably, it is dissolved in acetonitrile solution.

If required, the obtained substance can be purified through one of the conventional purification methods such as anti-solvent crystallization, activated charcoal crystallization, thin layer chromatography (TLC), column chromatography, high pressure liquid chromatography (HPLC), distillation and the methods in the prior art.

The method for the preparation of tiotropium bromide which is illustrated in formula 6 is comprised of the stages of dissolving quaternized scopine ester demonstrated in formula 5 in an oxygen-saturated organic solvent, for example in a solvent that is comprised of dimethylformamide (DMF), dimethyl sulfoxide (DMSO), benzene, toluene, diethyl ether, tetrahydrofurane, ethanol, methanol, acetonitrile, acetone, ethyl acetate, methyl ethyl ketone, dichloromethane, dioxane, dimethylacetamide, N-methyl pyrrolidone, hexane, heptane or a combination thereof, preferably in acetonitrile or dichloromethane; adding an organic or an inorganic base such as potassium carbonate, sodium carbonate, potassium hydrogen carbonate, sodium tert-butoxide, triethyl amine into the obtained solvent; and stirring the obtained mixture at -78-70 °C, preferably at -30-60 °C for 1-72 hours, preferably for 3-48 hours.

If required, the obtained substance can be purified through one of the conventional purification methods such as anti-solvent crystallization, activated charcoal crystallization, thin layer chromatography (TLC), column chromatography, high pressure liquid chromatography (HPLC), distillation and the methods in the prior art.

According to another aspect, the present invention comprises di-(2-thienyl)-acetic acid anhydride shown in formula 2 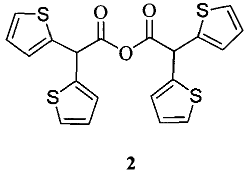 and the use of this substance in the synthesis of tiotropium bromide.

The synthesis method pertaining to the present invention can be explained with, but not limited to the following examples.

### Example 1: Process for the preparation of di-(2-thienyl)-acetic acid anhydride (2)

Di-(2-thienyl)-acetic acid (1) is dissolved in anhydrous CH₂Cl_{2;} the obtained mixture is added DCC and stirred for 24 hours at room temperature. The solid precipitate which comes out at the end is filtered and separated. The solvent in the obtained solution is removed at room temperature and under low pressure, and di-(2-thienyl)-acetic acid anhydride (2) is obtained (78% efficacy).

### Example 2: Process for the preparation of scopine ester (4)

Di-(2-thienyl)-acetic acid anhydride (2) and scopine are dissolved in anhydrous CH₂Cl_{2;} the obtained mixture is added DMAP and stirred for 24 hours at room temperature. Distilled water is added into the reaction mixture and it is stirred for 2 hours at room temperature. The obtained mixture is first diluted with CH₂Cl₂, then extracted with aqueous solutions 1M NaHSO₄, 10% Na₂CO₃ and saturated NaCl respectively. The organic part is separated and dried with anhydrous Na₂SO₄. CH₂Cl₂ in the solution obtained is removed under low pressure at room temperature. The obtained substance is purified through column chromatography and scopine ester **(4)** is obtained (84% efficacy).

### Example 3: Process for the preparation of quaternized scopine ester (5)

Scopine ester **(4)** and acetonitrile solution which comprises 50% of methyl bromide are dissolved in acetonitrile, and the obtained mixture is stirred for 72 hours at room temperature. The solid precipitate which comes out at the end is filtered, washed with an appropriate solvent, dried under low pressure and quartenized scopine ester **(5)** is obtained (94 % efficacy).

### Example 4: Process for the preparation of tiotropium bromide (6)

Quaternized scopine ester (5) and an organic base are dissolved in oxygen-saturated acetonitrile and the obtained mixture is stirred at room temperature for 48 hours. The solid precipitate which comes out at the end is filtered, washed with an appropriate solvent such as acetonitrile, dried under low pressure and tiotropium bromide **(6)** is obtained (74 % efficacy).

## Claims

1. A synthesis method so as to be used in the synthesis of tiotropium bromide (formula 6), 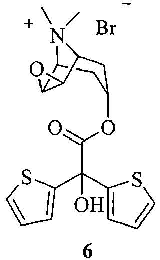 wherein said method comprises transforming di-(2-thienyl)-acetic acid 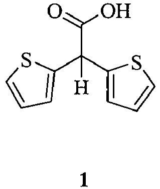 into Formula 2; 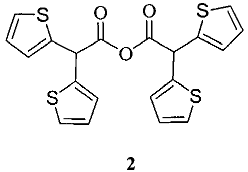 obtaining scopine ester shown in formula 4 as a consequence of the reaction of the obtained acid anhydride with scopine; 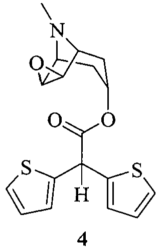 Obtaining the compound demonstrated in formula 5 after the obtained scopine ester is quaternized with methyl bromide; 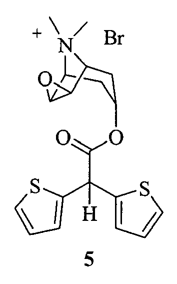 then processing this compound obtained at the end with organic and/or inorganic basic substances and transforming it into tiotropium bromide.

2. The synthesis method to be used in the synthesis of tiotropium bromide according to claim 1, wherein the method to be used in the synthesis of scopine ester shown in formula 4 comprises 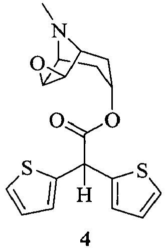 obtaining di-(2-thienyl)-acetic acid anhydride shown in formula 2 through the reaction of di-(2-thienyl)-acetic acid (1) with DCC; 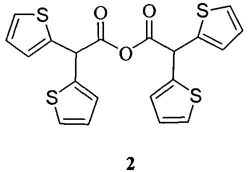 then obtaining scopine ester shown in formula 4 through the reaction of formula 2 and scopine with 4-dimethylaminopyridine (DMAP).

3. The method for the preparation of scopine ester illustrated in formula 4 according to claim 2, wherein the method for the synthesis of di-(2-thienyl)-acetic acid anhydride shown in formula 2 comprises the steps of dissolving di-(2-thienyl)-acetic acid (1) in an appropriate organic solvent; and adding N,N'-dicyclohexylcarbodiimide (DCC) into the obtained solution.

4. The method for the synthesis of di-(2-thienyl)-acetic acid anhydride shown in formula 2 according to claim 3, wherein at least 0,5 mole DCC is used per mole of di-(2-thienyl)-acetic acid in said reaction.

5. The method for the synthesis of di-(2-thienyl)-acetic acid anhydride shown in formula 2 according to claim 3, wherein the byproduct produced as a consequence of said reaction is separated by filtration method.

6. The method for obtaining scopine ester shown in formula 4 according to claim 2, wherein said method comprises the steps of dissolving scopine and di-(2-thienyl)-acetic acid anhydride in an appropriate organic solvent, then adding DMAP into the obtained solution.

7. The method for obtaining scopine ester shown in formula 4 according to claim 6, wherein the amount of di-(2-thienyl)-acetic acid anhydride used per mole of scopine is at least 1 mole, in said reaction.

8. The method for obtaining scopine ester shown in formula 4 according to claim 6, wherein the amount of DMAP used per mole of scopine is at least 0,05 mole in said reaction.

9. The method for obtaining scopine ester shown in formula 4 according to claim 6, wherein said reaction mixture is diluted with an appropriate organic solvent at the end of the reaction and it is extracted with the aqueous solutions 1M NaHSO₄, 10% Na₂CO₃ and saturated NaCl respectively.

10. The synthesis method according to claim 1, wherein the method to be used for the preparation of quaternized scopine ester shown in formula 5 comprises the steps of dissolving scopine ester shown in formula 4 and the organic solution which comprises 10-90% of methyl bromide in an appropriate solvent.

11. The method for the preparation of formula 5 according to claim 10, wherein the methyl bromide solvent used is prepared by condensing methyl bromide gas at low temperature, then dissolving it in a solvent combination which is comprised of dimethylformamide (DMF), dimethyl sulfoxide (DMSO), benzene, toluene, diethyl ether, tetrahydrofurane, ethanol, methanol, acetonitrile, acetone, ethyl acetate, methyl ethyl ketone, dichloromethane, dioxane, dimethylacetamide, N-methyl pyrrolidone, hexane, heptane or a combination thereof.

12. The synthesis method according to claim 1, wherein the method to be used for the preparation tiotropium bromide shown in formula 6 comprises the steps of dissolving quaternized scopine ester shown in formula 5 in a oxygen-saturated organic solvent, and adding an organic or inorganic base into the obtained solution.

13. The method to be used for the preparation of tiotropium bromide shown in formula 6 according to claim 12, wherein the organic or inorganic base used is chosen from a group comprising potassium carbonate, sodium carbonate, potassium hydrogen carbonate, sodium hydrogen carbonate, sodium tert-butoxide, triethyl amine.

14. The synthesis method according to Claim 9, wherein the organic solvent is chosen from a group comprising dimethylformamide (DMF), dimethyl sulfoxide (DMSO), benzene, toluene, diethyl ether, tetrahydrofurane, ethanol, methanol, acetonitrile, acetone, ethyl acetate, methyl ethyl ketone, dichloromethane, dioxane, dimethylacetamide, N-methyl pyrrolidone, hexane, heptane or a combination thereof.

15. The use of the compound of Formula 2 in the synthesis of tiotropium bromide

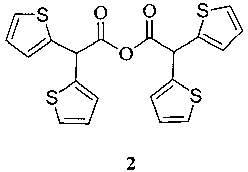

## Patentansprüche

1. Ein Syntheseverfahren, um bei der Synthese von Tiotropiumbromid (Formel 6) eingesetzt zu werden, 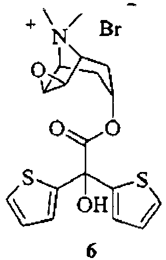 wobei das genannte Verfahren Umwandlung der di-(2-thienyl) -Essigsäure 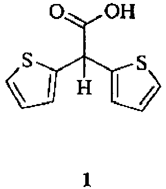 in Formel 2 umfasst; 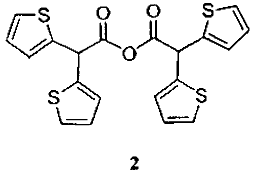 erhaltend Scopinester, der in Formel 4 als Folge der Reaktion des erhaltenen Säureanhydrids mit Scopin gezeigt wird; 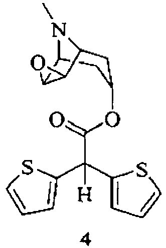 erhaltend die Verbindung, die in Formel 5 gezeigt wird, nachdem der erhaltene Scopinester mit Methylbromid quaternisiert wird; 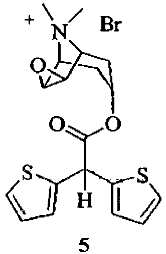 dann die Verarbeitung dieser Verbindung, die am Ende mit organischen und / oder anorganischen Grundstoffen erhalten wird und deren Umwandlung in Tiotropiumbromid.

2. Das Syntheseverfahren nach Anspruch 1, um bei der Synthese von Tiotropiumbromid eingesetzt zu werden, wobei das Verfahren, das bei der Synthese von in Formel 4 gezeigten Scopinester eingesetzt wird, 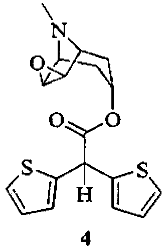 das Erhalten von di-(2-thienyl)-Essigsäureanhydrid umfasst, das in Formel 2 durch die Reaktion von di-(2-thienyl)-Essigsäure (1) mit DCC gezeigt wird; 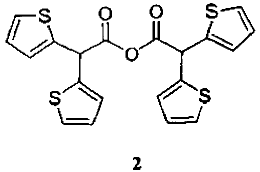 dann das Erhalten von Scopinester, der in Formel 4 durch die Reaktion der Formel 2 und Scopin mit 4-Dimethylaminopyridin (DMAP) gezeigt wird.

3. Das Verfahren zur Herstellung von Scopinester, der in Formel 4 nach Anspruch 2 dargestellt wird, wobei das Verfahren für die Synthese von in Formel 2 gezeigten di-(2-thienyl)-Essigsäureanhydrid die Schritte des Lösens der di-(2-thienyl)-Essigsäure (1) in einem geeigneten organischen Lösungsmittel und des Hinzufügens von N, N'-Dicyclohexylcarbodiimid (DCC) in die erhaltene Lösung umfasst.

4. Das Verfahren für die Synthese von di-(2-thienyl)-Essigsäureanhydrid, das in Formel 2 nach Anspruch 3 gezeigt wird, wobei mindestens 0,5 Mol von DCC pro Mol der di-(2-thienyl)-Essigsäure bei der genannten Reaktion verwendet wird.

5. Das Verfahren für die Synthese von di-(2-thienyl)-Essigsäureanhydrid, das in Formel 2 nach Anspruch 3 gezeigt wird, wobei das Nebenprodukt als Folge der genannten Reaktion durch Filtrationsverfahren abgetrennt wird.

6. Das Verfahren zum Erhalten von Scopinester, der in Formel 4 nach Anspruch 2 gezeigt wird, wobei das genannte Verfahren die Schritte des Lösens von Scopin und di-(2-thienyl)-Essigsäureanhydrid in einem geeigneten organischen Lösungsmittel und dann des Hinzufügens von DMAP in die erhaltene Lösung umfasst.

7. Das Verfahren zum Erhalten von Scopinester, der in Formel 4 nach Anspruch 6 gezeigt wird, wobei die Menge von di-(2-thienyl)-Essigsäureanhydrid, das pro Mol des Scopins bei der Reaktion verwendet wird, mindestens 1 Mol beträgt.

8. Das Verfahren zum Erhalten von Scopinester, der in Formel 4 nach Anspruch 6 gezeigt wird, wobei die Menge von DMAP, das pro Mol des Scopins verwendet wird, mindestens 0.05 Mol bei der genannten Reaktion beträgt.

9. Das Verfahren zum Erhalten von Scopinester, der in Formel 4 nach Anspruch 6 gezeigt wird, wobei das erwähnte Reaktionsgemisch mit einem geeigneten organischen Lösungsmittel am Ende der Reaktion verdünnt wird und mit den wäßrigen Lösungen in 1M NaHSO₄, 10% Na₂CO₃ und von gesättigtem NaCI jeweils extrahiert wird.

10. Das Syntheseverfahren nach Anspruch 1, wobei das Verfahren zur Herstellung von quaternisierten Scopinester, der in Formel 5 gezeigt wird, die Schritte des Lösens von in Formel 4 gezeigten Scopinester und die organische Lösung umfasst, die 10-90% von Methylbromid in einem geeigneten Lösungsmittel enthält.

11. Das Verfahren zur Herstellung der Formel 5 nach Anspruch 10, wobei das verwendete Methylbromid-Lösungsmittel durch Kondensieren von Methylbromid-Gas bei niedriger Temperatur und dann durch Auflösen in einer Lösungsmittelkombination hergestellt wird, bestehend aus Dimethylformamid (DMF), Dimethylsulfoxid (DMSO), Benzol, Toluol, Diethylether, Tetrahydrofuran, Ethanol, Methanol, Acetonitril, Aceton, Ethylacetat, Methylethylketon, Dichlormethan, Dioxan, Dimethylacetamid, N-Methylpyrrolidon, Hexan, Heptan oder eine Kombination davon.

12. Das Syntheseverfahren nach Anspruch 1, wobei das Verfahren zur Herstellung von Tiotropiumbromid, das in Formel 6 gezeigt wird, die Schritte des Auflösens des quaternisierten Scopinesters, das in Formel 5 in einem sauerstoffgesättigten organischen Lösungsmittel gezeigt wird, und eines Hinzufügens einer organischen oder anorganischen Base in die erhaltene Lösung umfasst.

13. Das Verfahren zur Herstellung von Tiotropiumbromid, das nach Anspruch 12 in Formel 6 gezeigt wird, wobei die verwendete organische oder anorganische Base aus einer Gruppe bestehend aus Kaliumcarbonat, Natriumcarbonat, Kaliumhydrogencarbonat, Natriumhydrogencarbonat, Natriumter-Butoxid und Triethylamin ausgewählt wird.

14. Das Syntheseverfahren nach Anspruch 9, wobei das organische Lösungsmittel aus einer Gruppe bestehend aus Dimethylformamid (DMF), Dimethylsulfoxid (DMSO), Benzol, Toluol, Diethylether, Tetrahydrofuran, Ethanol, Methanol, Acetonitril, Aceton, Ethylacetat, Methylethylketon, Dichlormethan, Dioxan, Dimethylacetamid, N-Methylpyrrolidon, Hexan, Heptan oder eine Kombination davon ausgewählt wird.

15. Die Verwendung der Verbindung der Formel 2 in der Synthese von Tiotropiumbromid

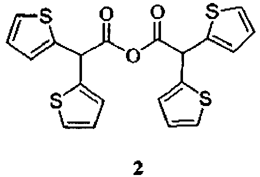

## Revendications

1. Méthode de synthèse pour être utilisée dans la synthèse du bromure de tiotropium (formule 6) 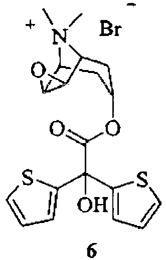 dans lequel ladite méthode comprend la transformation de di- (2-thiényl) -acide acétique 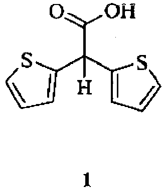 dans Formule 2; 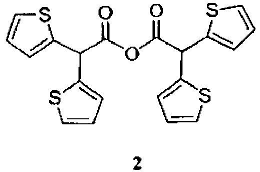 l'obtention d'un ester de scopine représenté dans la formule 4 en conséquence de la réaction de l'anhydride d'acide obtenu avec scopine; 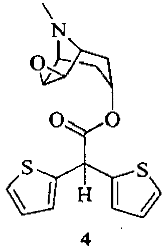 L'obtention du composé démontré dans la formule 5 après l'ester de scopine obtenu est quaternisé avec du bromure de méthyle; 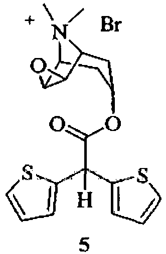 puis le traitement de ce composé obtenu à la fin avec des substances esentielles organiques et / ou inorganiques et le transformer en le bromure de tiotropium.

2. La méthode de synthèse à utiliser dans la synthèse du bromure de tiotropium selon la revendication 1, dans lequel la méthode à utiliser dans la synthèse de l'ester de scopine représenté dans la formule 4 comprend 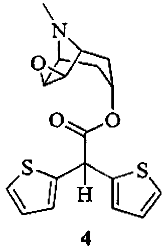 l'obtention de di- (2-thiényl) d'anhydride d'acide acétique représenté dans la formule 2 par la réaction de di-(2-thiényl) -acide acétique (1) avec du DCC 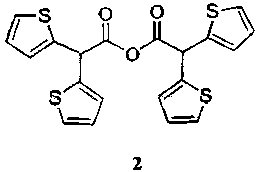 puis l'obtention d'un ester de scopine représenté dans la formule 4 par la réaction de formule 2 et scopine avec du 4-diméthylaminopyridine (DMAP).

3. La méthode pour la préparation d'un ester de scopine représenté dans la formule 4 selon la revendication 2, dans lequel la méthode pour la synthèse de di- (2-thiényl) d'anhydride d'acide acétique représenté dans la formule 2 comprend les étapes consistant à dissoudre le di- (2-thiényl ) acide acétique (1) dans un solvant organique approprié; et l'ajout de N, N'-dicyclohexylcarbodiimide (DCC) dans la solution obtenue.

4. La méthode pour la synthèse de di- (2-thiényl) d'andride d'acide acétique représentée dans la formule 2 selon la revendication 3, dans lequel au moins 0,5 mole de DCC est utilisé par mole de di- (2-thiényl) - acide acétique ladite réaction.

5. La méthode pour la synthèse de di- (2-thiényl) d'anhydride d'acide acétique représentée dans la formule 2 selon la revendication 3, dans lequel le sous-produit, produit en tant que conséquence de cette réaction est séparé par une méthode de filtration.

6. La méthode d'obtention d'un ester de scopine représentée dans la formule 4 selon la revendication 2, dans lequel ladite méthode comprend les étapes consistant à dissoudre scopine et le di- (2-thiényl) d'anhydride d'acide acétique dans un solvant organique approprié, puis l'addition de DMAP dans la solution obtenue.

7. La méthode d'obtention d'un ester de scopine représentée dans la formule 4 selon la revendication 6, dans lequel la quantité de di- (2-thiényl) d'anhydride d'acide acétique par mole de scopine est d'au moins 1 mole, dans ladite réaction.

8. La méthode d'obtention d'un ester de scopine représentée dans la formule 4 selon la revendication 6, dans lequel la quantité de DMAP utilisée par mole de scopine est d'au moins 0,05 molaire dans ladite réaction.

9. La méthode d'obtention d'un ester de scopine représentée dans la formule 4 selon la revendication 6, dans lequel ledit mélange réactionnel est dilué avec un solvant organique approprié à la fin de la réaction, et on l'extrait avec la solution aqueuse IM NaHSO₄,10% Na₂CO₃ et NaCl saturé, respectivement.

10. La méthode de synthèse selon la revendication 1, dans lequel la méthode à utiliser pour la préparation d'un ester de scopine quaternisé représenté dans la formule 5 comprend les étapes consistant à dissoudre l'ester de scopine représenté dans la formule 4 et la solution organique qui comprend 10 à 90% de bromure de méthyle dans un solvant approprié.

11. La méthode de la préparation de formule 5 selon la revendication 10, dans lequel le bromure de méthyle, le solvant utilisé est préparé par condensation du bromure de méthyle gazeux à basse température, puis le dissoudre dans un mélange solvant qui estconstitué de diméthylformamide (DMF), le diméthylsulfoxyde (DMSO ), le benzène, le toluène, l'éther diéthylique, tetrahydrofürane, l'éthanol, le méthanol, l'acétonitrile, l'acétone, l'acétate d'éthyle, la méthyl éthyl cétone méthyle, le dichlorométhane, le dioxane, le diméthylacétamide, la N-méthylpyrrolidone, l'hexane, l'heptane, ou une combinaison de ceux-ci.

12. La méthode de synthèse selon la revendication 1, dans lequel la méthode à utiliser pour la préparation de bromure de tiotropium représentée dans la formule 6 comprend les étapes consistant à dissoudre l'ester de scopine quaternisé représenté dans la formule 5 dans un solvant organique saturé en oxygène et ajouter une base organique ou inorganique, dans la solution obtenue.

13. La méthode à utiliser pour la préparation du bromure de tiotropium représentée dans la formule 6 selon la revendication 12, dans lequel la base organique ou inorganique utilisé est choisi d'un groupe comprenant le carbonate de potassium, le carbonate de sodium, le carbonate acide de potassium, le carbonate acide de sodium, le tert- de sodium butylate, le triéthyl amine.

14. La méthode de synthèse selon la revendication 9, dans lequel le solvant organique est choisi d'un groupe comprenant le diméthylformamide (DMF), le diméthylsulfoxyde (DMSO), le benzène, le toluène, l'éther diéthylique, tetrahydrofiirane, l'éthanol, le méthanol, l'acétonitrile, l'acétone, l'acétate d'éthyle, le méthyl éthyl cétone, le dichlorométhane, le dioxane, le diméthylacétamide, la N-méthylpyrrolidone, l'hexane, l'heptane, ou une combinaison de ceux-ci.

15. L'utilisation du composé de formule 2 dans la synthèse du bromure de tiotropium.
